# EUROPEAN PATENT APPLICATION

(11) **EP 4 105 333 A1**
(43) Date of publication of application: **21.12.2022**
(21) Application number: 21734043.9
(22) Date of filing: 24.05.2021
(51) Int. Cl.: C12N 15/77, C07K 14/34, C12P 13/08

(54) **NOVEL PROMOTER AND USE THEREOF**

(30) Priority: 07.05.2021 KR 20210059093
(71) Applicant: CJ Cheiljedang Corporation, Seoul 04560 (KR)
(72) Inventor: KWON, Nara, Seoul 04560 (KR); LEE, Han Hyoung, Seoul 04560 (KR); KIM, Hye Mi, Seoul 04560 (KR); PARK, Sojung, Seoul 04560 (KR); KIM, Byeong Soo, Seoul 04560 (KR)
(74) Representative: Icosa
(86) International application number: PCT/KR2021/006409
(87) International publication number: WO 2022/234881

(57) **Abstract**

Provided are a novel promoter and a method of producing a target material using the same. More particularly, provided are a novel polynucleotide having promoter activity, a gene expression cassette, and a host cell, each including the polynucleotide, and a method of producing a target material using the microorganism.

## Description

### BACKGROUND OF THE INVENTION

### 1. Field of the Invention

The present disclosure relates to a novel promoter and a method of producing a target material using the same. More particularly, the present disclosure relates to a novel polynucleotide having promoter activity, a vector, and a host cell, each including the polynucleotide, and a method of producing a target material using the microorganism.

### 2. Description of the Related Art

L-Amino acids are basic structural units of proteins and used as important materials for pharmaceutical raw materials, food additives, animal feeds, nutrients, pesticides, bactericides, *etc.* Among L-amino acids, L-lysine is an essential amino acid that is not biosynthesized in the living body, and is known to be necessary for growth promotion, calcium metabolism, promotion of gastric juice secretion, and resistance to diseases. L-Lysine is widely used in feeds, medical products, foods, *etc.* L-Tryptophan is also one of the essential amino acids, and used in feed additives, transfusion agents, pharmaceutical raw materials, health food materials, *etc.*

Common methods of producing the amino acids mainly include fermentation methods using microorganisms such as *Brevibacterium* or *Corynebacterium (Amino Acid Fermentation,* Gakkai Shuppan Center: 195-215, 1986), in addition to *Escherichia coli,* microorganisms of the genus *Bacillus, Streptomyces, Penicillium, Klebsiella, Erwinia, Pantoea, etc.* (U.S. Patent Nos. 3,220,929, and 6,682,912), and also include industrial methods through synthesis, such as a monochloroacetate method, a Strecker method, *etc.*

Further, many studies for efficient production of amino acids, for example, efforts to develop a microorganism with high amino acid production efficiency or a fermentation technology, have been made. Specifically, target material-specific approaches to increase expression of genes encoding enzymes involved in amino acid biosynthesis or to remove unnecessary genes in amino acid biosynthesis in a strain of the genus *Corynebacterium* have been developed (Korean Patent Nos. 10-0924065 and 1208480). In addition to these methods, a method of deleting genes which are not involved in the amino acid production and a method of deleting genes of which specific functions in the amino acid production are not known are also used. However, there is still a demand for research on a method capable of efficiently producing L-amino acids with a high yield.

In view of this technical background, the present inventors have developed a novel polynucleotide having promoter activity, which is able to produce a target material with high productivity in a microorganism of the genus *Corynebacterium,* and they found that when this polynucleotide is introduced into the microorganism, productivity of the target material may be increased, thereby completing the present disclosure.

The present inventors have made intensive efforts to prepare a novel polynucleotide having promoter activity, and as a result, they found that when a target gene promoter is improved through base substitution, the improved promoter is able to regulate expression of a gene operably linked thereto, thereby completing the present disclosure.

### SUMMARY OF THE INVENTION

An object of the present disclosure is to provide a polynucleotide having promoter activity, in which a nucleotide at position 268 of a nucleotide sequence represented by SEQ ID NO: 1 is substituted with G.

Another object of the present disclosure is to provide a gene expression cassette including the polynucleotide and a target gene.

Still another object of the present disclosure is to provide a host cell including the polynucleotide or the gene expression cassette.

Still another object of the present disclosure is to provide a method of producing a target material, the method including the steps of culturing the host cell in a medium; and recovering the target material from the medium.

Still another object of the present disclosure is to provide use of a polynucleotide as a promoter, in which a nucleotide at position 268 of a nucleotide sequence represented by SEQ ID NO: 1 is substituted with another nucleotide.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or will be able to ascertain using no more than routine experimentation, many equivalents to the specific embodiments of the disclosure described herein. Further, these equivalents should be interpreted to fall within the present disclosure.

To achieve the above objects, an aspect of the present disclosure provides a polynucleotide having promoter activity, in which a nucleotide at position 268 of a nucleotide sequence represented by SEQ ID NO: 1 is substituted with G.

As used herein, the term "nucleotide sequence represented by SEQ ID NO: 1" may refer to a part of a promoter sequence of NCgl1416 gene.

In this regard, the term "NCgl1416 gene" refers to a gene that is inherently present in a microorganism of the genus *Corynebacterium* or a gene encoding a hypothetical protein whose function is not known.

The sequence corresponding to SEQ ID NO: 1 may be derived from *Corynebacterium* (*Corynebacterium* sp.), and specifically a sequence derived from *Corynebacterium glutamicum.* However, a sequence having activity equivalent to or greater than that of the polynucleotide may be included in the promoter of the present disclosure without limitation.

As used herein, the term "polynucleotide", which refers to a nucleotide polymer in which nucleotide monomers are covalently bonded in a long chain, refers to a DNA strand having a predetermined length or more.

As used herein, the term "promoter" refers to an untranslated nucleotide sequence upstream of a coding region, which includes a binding site for polymerase and has the activity of initiating transcription of a promoter target gene into mRNA, *i.e.,* a DNA domain to which polymerase binds to initiate the transcription of a gene. The promoter may be located at the 5' domain of an mRNA transcription initiation domain.

As used herein, the term "polynucleotide having promoter activity" refers to a DNA region which includes a binding site for RNA polymerase or enhancer, *etc.* for expression of a gene operably linked downstream thereof, *i.e*., a target gene, and exists near a transcription site of the target gene. With respect to the objects of the present disclosure, the polynucleotide may be used as a general-use promoter, and as compared with the existing promoter or endogenous promoter in cells, the polynucleotide may regulate (e.g., increase or decrease) expression of a target gene operably linked thereto and production and/or activity of a protein encoded by the target gene, and it may regulate (e.g., increase or decrease) production and/or activity of a target product (a biologically active material, for example, one or more selected from the group consisting of amino acids, nucleic acids, vitamins, proteins, fatty acids, organic acids, *etc*.) involved in the production of the protein, but is not limited thereto.

The polynucleotide of the present disclosure may include any polynucleotide sequence without limitation, as long as it has the promoter activity.

In one embodiment, the polynucleotide having promoter activity of the present disclosure may include a polynucleotide having promoter activity, in which one or more nucleotides in the nucleotide sequence represented by SEQ ID NO: 1 are substituted with another nucleotide. Specifically, the polynucleotide may consist of a polynucleotide having promoter activity, in which one or more nucleotides in the nucleotide sequence of SEQ ID NO: 1 are substituted with another nucleotide. The polynucleotide having promoter activity may be used interchangeably with a "variant promoter" in the present disclosure, and all of the above-described terms may be used in the present disclosure.

For example, the polynucleotide having promoter activity may be a polynucleotide in which a nucleotide at a specific position of a polynucleotide sequence having existing promoter activity is substituted, and thus the promoter activity is weakened or enhanced.

In one embodiment, the variant promoter may be a promoter in which a nucleotide at position 268 of the nucleotide sequence represented by SEQ ID NO: 1 is substituted with another nucleotide.

The "other nucleotide" is not limited, as long as it is a nucleotide different from the nucleotide before substitution. For example, when it is described that "the nucleotide at position 268 of SEQ ID NO: 1 is substituted with another nucleotide", it means that the nucleotide is substituted with thymine (T), cytosine (C), or guanine (G) except for adenine (A). Also, unless indicated otherwise, when a nucleotide is described as being "substituted" in the present disclosure, it means that the nucleotide is substituted with a nucleotide different from the nucleotide before substitution.

Meanwhile, those skilled in the art may identify a nucleotide at the position which corresponds to position 268 of SEQ ID NO: 1 of the present disclosure in any polynucleotide sequence through sequence alignment known in the art. Unless otherwise described in the present disclosure, when "a nucleotide at a specific position of a specific SEQ ID NO" is described, it is apparent that the nucleotide also includes "a nucleotide at the corresponding position" in any polynucleotide sequence. Therefore, a polynucleotide sequence in which any one or more nucleotides selected from the group consisting of nucleotides at positions corresponding to position 268 of the polynucleotide sequence of SEQ ID NO: 1 are substituted with another nucleotide in any polynucleotide sequence having promoter activity is also included in the scope of the present disclosure.

The polynucleotide of the present disclosure may be a polynucleotide in which the nucleotide sequence represented by SEQ ID NO: 1, *i.e.,* the promoter sequence of NCgl1416 gene, is altered. Specifically, the alteration may be substitution of the nucleotide at position 268 of the sequence with another nucleotide. Specifically, the nucleotide at position 268 of the nucleotide sequence represented by SEQ ID NO: 1 may be substituted with G.

The polynucleotide may have altered (increased or decreased) promoter activity, as compared with a polynucleotide without the alteration. Therefore, expression of a target gene operably linked to the polynucleotide and activity of a protein encoded by the target gene may be regulated (increased or decreased), and furthermore, expression of a gene other than the target gene may be regulated.

For example, when the nucleotide at position 268 of the nucleotide sequence represented by SEQ ID NO: 1 is substituted with another nucleotide, it is possible to provide a promoter having activity which is more weakened or enhanced than the unsubstituted (unaltered) promoter sequence.

Further, the polynucleotide may be a polynucleotide involved in increasing production or a production amount of a target material, specifically, an L-amino acid, and more specifically L-lysine.

The effect of increasing the production amount of an L-amino acid, more specifically L- lysine, by the promoter alteration of NCgl1416 gene has been investigated for the first time by the present inventors.

As used herein, the "L-lysine", which is one of basic α-amino acids, is an essential amino acid not synthesized *in vivo.* The L-lysine may be applied to, but is not limited to, various products such as feeds or feed additives, or foods, food additives, medical products, *etc.*

In one embodiment, the polynucleotide having promoter activity of the present disclosure may include a polynucleotide sequence of SEQ ID NO:2. Specifically, the polynucleotide may essentially consist of or may consist of the polynucleotide sequence of SEQ ID NO: 2, but is not limited thereto.

In addition, without limitation to the above-described embodiment, various alterations may be included in the polynucleotide sequence within a range that does not significantly reduce the promoter activity. For example, the nucleotide sequence of the present disclosure may be altered by way of a known mutagenesis method, for example, directed evolution, site-directed mutagenesis, *etc.*

In this regard, the term "alteration" refers to a genetically or non-genetically stable phenotypic change, and in the present disclosure, it may be used interchangeably with "modification" or "mutation".

Therefore, in the present disclosure, the polynucleotide having promoter activity may be a polynucleotide sequence of SEQ ID NO: 1 or SEQ ID NO: 2, or a polynucleotide sequence having at least 70%, 80%, 85%, 90%, 95%, 96%, 97%, 98%, or 99% or more homology or identity to SEQ ID NO: 1 or SEQ ID NO: 2. The nucleotide sequence having homology or identity may be a sequence having less than 100% identity, except for a sequence having 100% identity among the above categories.

Meanwhile, although described as "a polynucleotide having a nucleotide sequence represented by a particular SEQ ID NO" or "a polynucleotide including a nucleotide sequence represented by a particular SEQ ID NO" in the present disclosure, it is obvious that a polynucleotide having a nucleotide sequence with deletion, modification, substitution, or addition in part of the sequence may also be used in the present disclosure, as long as it has activity identical or corresponding to that of the polynucleotide consisting of the nucleotide sequence of the corresponding SEQ ID NO.

For example, it is obvious that a polynucleotide in which a meaningless sequence is added to the inside or end of the nucleotide sequence of the corresponding SEQ ID NO, or in which a part of the sequence is deleted in the inside or end of the nucleotide sequence of the corresponding SEQ ID NO, is also included in the scope of the present disclosure, as long as it has activity identical or corresponding to that of the polynucleotide.

Homology or identity refers to a degree of matching with two given nucleotide sequences, and may be expressed as a percentage.

The terms "homology" and "identity" may often be used interchangeably.

The sequence homology or identity of conserved polynucleotides may be determined using standard alignment algorithms and may be used with a default gap penalty established by the program being used. Substantially homologous or identical sequences may hybridize under moderate or high stringency, along the entire length or at least about 50%, about 60%, about 70%, about 80%, or about 90% of the entire length of the sequences. Polynucleotides that contain degenerate codons instead of codons in the hybridizing polynucleotides are also considered.

Whether any two polynucleotide sequences have homology, similarity, or identity may be determined using a known computer algorithm such as the "FASTA" program using a default parameter, for example, as in Pearson et al. (1988) Proc. Natl. Acad. Sci. USA 85:2444. Alternatively, as performed in the Needleman program of the EMBOSS package (EMBOSS: The European Molecular Biology Open Software Suite, Rice et al., 2000, Trends Genet. 16:276-277) (version 5.0.0 or subsequent versions), homology, similarity, or identity may be determined using the Needleman-Wunsch algorithm (Needleman and Wunsch, 1970, J. Mol. Biol. 48:443-453) (including the GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, homology, similarity, or identity may be determined using BLAST or ClustalW of the National Center for Biotech Information Database.

Homology, similarity, or identity of polynucleotides may be determined by comparing sequence information using a GAP computer program, for example, Needleman et al. (1970), J Mol Biol. 48:443, as disclosed in Smith and Waterman, Adv. Appl. Math (1981) 2:482. In summary, the GAP program is defined as a value obtained by dividing the number of similar arranged symbols (*i.e.,* nucleotides or amino acids) by the entire number of symbols in the shorter of the two sequences. The default parameter for the GAP program may include (1) a binary comparison matrix (containing values of 1 for identity and 0 for non-identity) and a weighted comparison matrix of Gribskov et al. (1986) Nucl. Acids Res. 14:6745, as disclosed in Schwartz and Dayhoff, eds., Atlas Of Protein Sequence And Structure, National Biomedical Research Foundation, pp. 353-358 (1979) (alternatively, a substitution matrix of EDNAFULL (EMBOSS version of NCBI NUC4.4); (2) 3.0 penalty for each gap and additional 0.10 penalty for each symbol in each gap (alternatively, gap opening penalty 10, gap extension penalty 0.5); and (3) non- penalty for a terminal gap. Accordingly, the term "homology" or "identity" used in the present disclosure represents relatedness between sequences.

In addition, a probe which may be prepared from a known gene sequence, for example, a polynucleotide sequence having the same activity and hybridizing with a sequence complementary to the entirety or a part of the above-described polynucleotide sequence under stringent conditions may be included without limitation. The "stringent conditions" mean conditions which allow specific hybridization between polynucleotides.These conditions are specifically disclosed in the literature (e.g., J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). For example, the stringent conditions may include conditions under which genes having high homology or identity, genes having 40% or more, specifically 70% or more, 80% or more, 85% or more, or 90% or more, more specifically 95% or more, much more specifically 97% or more, and particularly specifically 99% or more homology or identity hybridize with each other, while genes having homology or identity lower than the above homology do not hybridize with each other; or may include ordinary washing conditions of Southern hybridization, *i.e.,* washing once, specifically two or three times, at a salt concentration and a temperature corresponding to 60°C, 1×SSC, and 0.1% SDS, specifically 60°C, 0.1×SSC, and 0.1% SDS, and more specifically 68°C, 0.1×SSC, and 0.1% SDS.

Hybridization requires that two nucleic acids have complementary sequences, although mismatches between nucleotides may be possible depending on the stringency of the hybridization. The term "complementary" is used to describe the relationship between mutually hybridizable nucleotides. For example, with respect to DNA, adenine is complementary to thymine, and cytosine is complementary to guanine. Therefore, the present disclosure may also include an isolated nucleic acid fragment complementary to the entire sequence as well as a nucleic acid sequence substantially similar thereto.

Specifically, a polynucleotide having homology or identity may be detected using hybridization conditions including a hybridization step at a Tₘ value of 55°C under the above-described conditions. Further, the Tₘ value may be 60°C, 63°C, or 65°C, but is not limited thereto, and may be appropriately controlled by those skilled in the art depending on the purpose thereof.

Stringency suitable for the hybridization of polynucleotides depends on the length and complementarity of the polynucleotides, and variables are well known in the art (see Sambrook *et al., supra,* 9.50-9.51, 11.7-11.8).

The polynucleotide having promoter activity of the present disclosure may be isolated or prepared using standard molecular biology techniques. For example, the polynucleotide may be prepared using the standard synthesis technology which uses an automated DNA synthesizer, but the preparation is not limited thereto.

The polynucleotide having promoter activity of the present disclosure may be used as a promoter.

The promoter may be located at the 5'-region of the mRNA transcription initiation site.

The promoter may have increased or decreased promoter activity, as compared with existing promoters. In other words, the promoter may increase or decrease expression and/or activity of a protein encoded by a target gene as well as expression of the target gene in host cells. With respect to the objects of the present disclosure, the target gene of which expression is enhanced or weakened may be changed according to a product to be produced, and the promoter may be used as a general-use promoter for enhancing or weakening the target gene.

Another aspect of the present disclosure provides a gene expression cassette including the polynucleotide and a target gene.

The polynucleotide of the present disclosure is the same as described above.

As used herein, the term "gene expression cassette" refers to a unit cassette which includes a promoter and a target gene, and is capable of expressing the target gene operably linked downstream of the promoter. Various factors that are able to aid the efficient expression of the target gene may be included inside or outside of the gene expression cassette. Generally, the gene expression cassette may include a transcription termination signal, a ribosome-binding domain, and a translation termination signal, in addition to the promoter operably linked to the target gene, but is not limited thereto.

The "target gene" refers to a gene whose expression is regulated by the promoter sequence of the present disclosure with respect to the objects of the present disclosure. A protein encoded by the target gene may be expressed as a "target protein", and a gene encoding the "target protein" may be expressed as a "target gene".

Further, the polynucleotide encoding the target protein may have various modifications in the coding region within a range that does not change the polypeptide sequence, due to codon degeneracy or considering codons preferred by an organism to express the polynucleotide. Description of the polynucleotide sequence is the same as described above.

In particular, the expression "consisting of the nucleotide sequence of SEQ ID NO: 2" means that, as when using a restriction enzyme, addition, deletion, and/or alteration of the nucleotide are/is not excluded, which may occur during the process of ligating to a target gene when the corresponding polynucleotide is used as a promoter by ligating to the target gene.

Further, the polynucleotide having the promoter activity consisting of the nucleotide sequence represented by SEQ ID NO: 2 may include any nucleotide sequence without limitation, as long as it is a nucleotide sequence which is hybridized with a complementary sequence to all or a part of the nucleotide sequence of SEQ ID NO: 2 under stringent conditions to have the promoter activity of the present disclosure.

Still another aspect of the present disclosure provides a recombinant vector including the polynucleotide, or the gene expression cassette including the polynucleotide and the target gene.

The polynucleotide, target gene, and gene expression cassette of the present disclosure are the same as described above.

As used herein, the term "vector" refers to an artificial DNA molecule having a genetic material capable of expressing a target gene in a suitable host, and specifically refers to a DNA construct including a nucleotide sequence of a gene encoding a target protein operably linked thereto.

As used herein, the term "operably linked" means that the polynucleotide having promoter activity of the present disclosure is functionally linked to the gene sequence to initiate and mediate transcription of the target gene. Operable linking may be achieved using a gene recombination technique known in the art, and site-specific DNA cleavage and ligation may be performed by using a restriction enzyme and ligase known in the art, but these are not limited thereto.

The vector used in the present disclosure is not particularly limited as long as it may be expressed in a host cell, and any vector known in the art may be used to transform the host cell. Examples of the vector commonly used may include natural or recombinant plasmids, cosmids, viruses, and bacteriophages.

For example, as a phage vector or cosmid vector, pWE15, M13, MBL3, MBL4, IXII, ASHII, APII, t10, t11, Charon4A, Charon21A, *etc.* may be used; and as a plasmid vector, those based on pDZ, pBR, pUC, pBluescriptII, pGEM, pTZ, pCL, pET, *etc.* may be used, but the vectors are not limited thereto. Specifically, pDZ, pDC, pDCM2, pACYC177, pACYC184, pCL, pECCG117, pUC19, pBR322, pMW118, pCC1BAC vectors, *etc.* may be used, but the vectors are not limited thereto. Further, insertion of the polynucleotide into the chromosome may be achieved by way of any method known in the art, *e.g.,* homologous recombination.

Since the vector of the present disclosure may be inserted into the chromosome by inducing homologous recombination, a selection marker may be additionally included to confirm successful gene insertion into the chromosome. The selection marker is for screening cells transformed with the vector, *i.e.,* for determining whether the polynucleotide is inserted. The markers that provide selectable phenotypes such as drug resistance, auxotrophy, resistance to toxic agents, or expression of surface proteins may be used. In an environment treated with a selective agent, only the cells expressing the selection marker are able to survive or to exhibit a different phenotype, and thus the transformed cells may be selected.

As used herein, the term "transformation" refers to introduction of a vector including a polynucleotide encoding a target protein into a host cell in order to allow expression of a protein encoded by the polynucleotide in the host cell. Furthermore, as long as the transformed polynucleotide may be expressed in the host cell, it does not matter whether the transformed polynucleotide is located on the chromosome of the host cell or outside of the chromosome, and both cases are included. Further, the polynucleotide includes DNA and RNA which encode the target protein. The polynucleotide may be introduced in any form as long as it may be introduced into a host cell and expressed therein. For example, the polynucleotide may be introduced into a host cell in the form of an expression cassette, which is a gene construct including all elements necessary for selfexpression, or in the form of a vector including the same.

The transformation method includes any method of introducing the gene encoding the target protein into a cell, and may be performed by selecting a suitable standard technique known in the art according to the host cell. For example, the transformation method may include electroporation, calcium phosphate (CaPO₄) precipitation, calcium chloride (CaCl₂) precipitation, microinjection, a polyethylene glycol (PEG) technique, a DEAE- dextran technique, a cationic liposome technique, a lithium acetate-DMSO technique, *etc.,* but the method is not limited thereto.

Still another aspect of the present disclosure provides a host cell including the polynucleotide or the gene expression cassette.

Specifically, the host cell may be a microorganism of the genus *Corynebacterium,* but is not limited thereto.

The polynucleotide and the gene expression cassette are the same as described above.

As used herein, the term "microorganism" includes all of a wild-type microorganism and a naturally or artificially genetically modified microorganism, and it is a concept including a microorganism having a particular attenuated or reinforced mechanism due to insertion of a foreign gene or reinforcement or attenuation of activity of an endogenous gene. In the present disclosure, the microorganism may include any microorganism without limitation, as long as it is introduced with or includes the polynucleotide of the present disclosure.

In the present disclosure, the microorganism may include the polynucleotide, specifically the polynucleotide and/or a vector including the polynucleotide, and more specifically a vector including the gene encoding the target protein, but is not limited thereto. In addition, the polynucleotide and the vector may be introduced into the microorganism by transformation, but these are not limited thereto.

The microorganism is a cell or microorganism that is transformed with the vector including the polynucleotide of the present disclosure and a gene encoding a target protein to express the target protein, and with respect to the objects of the present disclosure, the host cell or microorganism may be any microorganism, as long as it is able to produce a target product including the target protein.

As used herein, the term "microorganism producing the target protein or target product" includes all of a wild-type microorganism and a naturally or artificially genetically modified microorganism, and it refers to a microorganism having a particular attenuated or reinforced mechanism due to insertion of a foreign gene or reinforcement or inactivation of activity of an endogenous gene. The microorganism may be a microorganism including a genetic modification for producing a target protein or product.

With respect to the objects of the present disclosure, the microorganism producing the target protein or target product may be a microorganism that has increased productivity of the target protein or target product by including the polynucleotide of the present disclosure. Specifically, in the present disclosure, the microorganism producing the target protein or target product, or the microorganism having productivity of the target protein or target product may be a microorganism in which some of the genes in the biosynthetic pathway of the target protein or target product are enhanced or attenuated, or some of the genes in the degradation pathway of the target protein or target product are enhanced or attenuated.

With respect to the objects of the present disclosure, the microorganism including the polynucleotide may have increased production of an L-amino acid, specifically L-lysine.

In the present disclosure, the microorganism may include any microorganism without limitation, as long as it is a microorganism into which the polynucleotide having the promoter activity of the present disclosure is introduced, and thus is able to operate as a promoter.

The microorganism may specifically be a microorganism of the genus *Corynebacterium,* more specifically *Corynebacterium glutamicum* or *Corynebacterium flavum,* and most specifically *Corynebacterium glutamicum,* but is not limited thereto.

Still another aspect of the present disclosure provides a method of producing a target material, the method including the steps of culturing the host cell in a medium; and recovering the target material from the medium.

The host cell is the same as described above.

In the present disclosure, the target material may be an amino acid. Specifically, the amino acid may be an L-type amino acid unless otherwise mentioned, and may be selected from the group consisting of glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, proline, and combinations thereof, but is not limited thereto.

More specifically, the amino acid may be L-lysine, but is not limited thereto.

As used herein, the term "culture" refers to growing a microorganism under appropriately and artificially controlled environmental conditions. In the present disclosure, the method of producing the target material using the microorganism including the polynucleotide may be performed by using a method widely known in the art. Specifically, the culture may be performed continuously in a batch, a fed batch, or a repeated fed batch process, but is not limited thereto. A medium used in the culture must appropriately satisfy the requirements of specific strains. Culture media for the microorganisms of the genus *Corynebacterium* are disclosed (*e.g.,* Manual of Methods for General Bacteriology by the American Society for Bacteriology, Washington D.C., USA, 1981).

As a carbon source to be used in the media, sugars and carbohydrates such as glucose, sucrose, lactose, fructose, maltose, starch, and cellulose; oils and fats such as soybean oil, sunflower oil, castor oil, coconut oil, *etc.;* fatty acids such as palmitic acid, stearic acid, and linoleic acid; alcohols such as glycerol and ethanol; and organic acids such as acetic acid may be included. These materials may be used individually or in a mixture, but are not limited thereto.

As a nitrogen source to be used, peptone, a yeast extract, a beef extract, a malt extract, a corn steep liquor, soybean meal powder, and urea or inorganic compounds such as ammonium sulfate, ammonium chloride, ammonium phosphate, ammonium carbonate, and ammonium nitrate may be included. The nitrogen sources may also be used individually or in a mixture, but are not limited thereto.

As a phosphorus source to be used, potassium dihydrogen phosphate or dipotassium hydrogen phosphate or corresponding sodium-containing salts may be used. In addition, the culture medium may include a metal salt such as magnesium sulfate or iron sulfate which is essential for growth. Lastly, essential materials for growth such as amino acids and vitamins may be used in addition to the above-mentioned materials. Additionally, appropriate precursors may be used in the culture medium. The above source materials may be adequately fed into the culture in a batch or continuous manner during the culture process.

During the culturing of the microorganism, pH of the culture may be properly adjusted using a basic compound such as sodium hydroxide, potassium hydroxide, or ammonia, or an acidic compound such as phosphoric acid or sulfuric acid. Further, foam generation may be prevented using an anti-foaming agent such as a fatty acid polyglycol ester. To maintain the aerobic condition, oxygen or oxygen-containing gas (*e.g.,* air) may be introduced into the culture.

The temperature of the culture (medium) may be generally 20°C to 45°C, specifically 25°C to 40°C. The culturing may be continued until a desired amount of the target material is obtained, but it may be specifically performed for 10 hours to 160 hours.

With regard to the recovering of the target material from the culture (medium), the target material may be separated and recovered by a common method known in the art. The separation method may employ centrifugation, filtration, chromatography, crystallization, *etc.* For example, a supernatant obtained by centrifuging the culture medium at a low speed and removing a biomass may be separated by ion-exchange chromatography, but is not limited thereto.

The recovering step may further include a purification process.

Still another aspect of the present disclosure provides use of a polynucleotide as a promoter, in which a nucleotide at position 268 of a nucleotide sequence represented by SEQ ID NO: 1 is substituted with another nucleotide.

The polynucleotide is the same as described above.

Hereinafter, the present disclosure will be described in more detail with reference to Examples. However, the following Examples are merely preferred embodiments for illustrating the present disclosure, and therefore are not intended to limit the scope of the present disclosure thereto. On the other hand, technical matters not described in this specification may be sufficiently understood and easily implemented by those skilled in the art or similar technical fields of the present disclosure.

### Example 1. Preparation of Recombinant Vector including Novel Promoter Sequence

First, based on the U.S. National Institutes of Health GenBank (NIH GenBank), a nucleotide sequence (SEQ ID NO: 1) including a promoter region of NCgl1416 of wild- type *Corynebacterium glutamicum* ATCC13032 was obtained. To examine the effect of a promoter variant (PNCgl 1416(a268g); SEQ ID NO: 2) on L-lysine production, the promoter variant in which a nucleotide corresponding to position 268 of a polynucleotide of SEQ ID NO: 1 was substituted from A to G, a vector for preparing a strain expressing the same was prepared using a plasmid pDCM2 (Korean Patent Publication No. 10-2020-0136813) for insertion and replacement of a gene in the chromosome of the *Corynebacterium.*

In detail, gDNA (genomic DNA) of the wild-type *Corynebacterium glutamicum* ATCC13032 as a template, and a pair of primers of sequences of SEQ ID NOs: 3 and 4 and a pair of primers of sequences of SEQ ID NOs: 5 and 6 were each used to perform PCR. A mixture of the two fragments obtained as above as a template and a pair of primers of sequences of SEQ ID NO: 3 and SEQ ID NO: 6 were used to perform overlapping PCR to thereby obtain a fragment. PCR was performed under the following conditions: denaturation at 94°C for 5 minutes; 30 cycles of 94°C for 30 seconds, 55°C for 30 seconds, and 72°C for 1 minute and 30 seconds; and 72°C for 5 minutes. The pDCM2 plasmid was treated with SmaI, and the obtained PCR product was fusion-cloned thereinto. In the fusion cloning, an In-Fusion^{®} HD cloning kit (Clontech) was used. The resulting vector was designated as pDCM2-PNCgl 1416(a268g). The information of the primer sequences used for the preparation of the vector is shown in Table 1 below.

**[Table 1]**

| SEQ ID NO | Primer name | 5' sequence 3' |
|---|---|---|
| 3 | PNCgl1416_1F | TGAATTCGAGCTCGGTACC CCCAGTTGCCAAGGTTGGC |
| 4 | PNCgl1416_2R | CACCTTGTCCGCCGCTcGA GAATTTTCCTCCGG |
| 5 | PNCgl1416_3F | CCGGAGGAAAATTCTCgAG CGGCGGACAAGGTG |
| 6 | PNCgl1416_4R | GTCGACTCTAGAGGATCCC CGTTGAGAGCCCAGCCGG |

### Example 2. Evaluation of L-lysine productivity of microorganism including novel promoter sequence

### 2-1. Preparation of PNCgl1416 variant-expressing strain

The gene-substituted vector prepared in Example 1 was introduced into a *Corynebacterium glutamicum* CJ3P (US 9556463 B2) strain to prepare a variant-introduced L-lysine-producing strain, "CJ3P_PNCgl1416(a268g)".

In detail, transformation was performed by electroporation (Appl. Microbiol. Biotechnol., 1999, 52:541-545), and then the strain, in which the vector was inserted into the chromosome by way of homologous sequence recombination, was selected on an agar nutrient medium containing 25 mg/L kanamycin. The primary selected strain was subjected to secondary crossover to select a target variant-introduced strain. The variation (substitution) in the final transformed strain was examined by PCR using a pair of primers of SEQ ID NOs: 7 and 8, followed by sequencing. The information of the primer sequences used for the preparation of the PNCgl1416 variant-expressing strain is shown in Table 2 below.

**[Table 2]**

| SEQ ID NO | Primer name | 5' sequence 3' |
|---|---|---|
| 7 | PNCgl1416_5F | CAGGATGGGGAAGTTGTC |
| 8 | PNCgl1416_6R | GACCACTGTACGCGAGG |

### 2-2. Comparison of L-lysine productivity of PNCgl1416 variant-expressing strain

L-Lysine productivity was analyzed by way of flask fermentation titer of the strain prepared in Example 2-1 and the control parent strain.First, each strain was inoculated in a 250 mL corner-baffle flask containing 25 mL of a seed medium, and shake-cultured at 30°C for 20 hours at 200 rpm. 1 mL of the seed culture solution was inoculated in a 250 mL corner-baffle flask containing 24 mL of a production medium, and shake-cultured at 30°C for 72 hours at 200 rpm. In this regard, compositions of the seed medium and the production medium were as follows.

### Seed medium (pH 7.0)

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 2 mg of calcium pantothenate, and 2 mg of nicotinamide (based on 1 L of distilled water)

### Production medium (pH 7.0)

100 g of glucose, 40 g of (NH₄)₂SO₄, 2.5 g of soybean protein, 5 g of corn steep solids, 3 g of urea, 1 g of KH₂PO₄, 0.5 g of MgSO₄.7H₂O, 100 µg of biotin, 1000 µg of thiamine HCl, 2000 µg of calcium pantothenate, 3000 µg of nicotinamide, and 30 g of CaCO₃ (based on 1 L of distilled water)

After the culture was terminated, L-lysine productivity was measured using HPLC, and the lysine concentration in the culture medium and the concentration increase rate for each strain are shown in Table 3 below.

**[Table 3]**

| Strain name | L-Lysine concentration (g/L) | Lysine concentration increase rate (%) |
|---|---|---|
| CJ3P | 7.87 | - |
| CJ3P_PNCgl1416(a268g) | 9.43 | 19.8 |

As shown in Table 3, it was confirmed that the variation-introduced *Corynebacterium glutamicum* CJ3P_PNCgl1416(a268g) strain exhibited a 19.8% increase in the L-lysine concentration, as compared with the *Corynebacterium glutamicum* CJ3P strain into which no variation was introduced.

In other words, the variation was confirmed to remarkably increase L-lysine productivity of the microorganism.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

### Effect of the invention

A novel promoter of the present disclosure is introduced into an amino acid-producing microorganism to increase the amino acid productivity of the microorganism. In particular, when an amino acid is produced using the novel promoter, L-lysine, which has thus far been produced by way of existing synthesis methods, may be produced by a fermentation method, and thus the promoter may be usefully applied to amino acid production.

## Claims

1. A polynucleotide having promoter activity, wherein a nucleotide at position 268 of a nucleotide sequence represented by SEQ ID NO: 1 is substituted with G.

2. The polynucleotide of claim 1, wherein the polynucleotide consists of a nucleotide sequence of SEQ ID NO: 2.

3. A gene expression cassette comprising the polynucleotide of claim 1 and a target gene.

4. A host cell comprising the polynucleotide of claim 1 or the gene expression cassette of claim **3**.

5. The host cell of claim **4**, wherein the host cell is a microorganism of the genus *Corynebacterium.*

6. The host cell of claim **5**, wherein the microorganism of the genus *Corynebacterium* is *Corynebacterium glutamicum.*

7. A method of producing a target material, the method comprising the steps of culturing the host cell of claim **4** in a medium; and recovering the target material from the medium.

8. The method of claim **7**, wherein the target material is an amino acid.

9. The method of claim **7**, wherein the target material is L-lysine.
